# EUROPEAN PATENT SPECIFICATION

(11) **EP 4 321 194 B1**
(45) Date of publication and mention of the grant of the patent: **08.10.2025**
(21) Application number: 23765129.4
(22) Date of filing: 31.05.2023
(51) Int. Cl.: A61M 15/00, A61M 11/02, A61M 15/06

(54) **INHALER**
INHALATOR
INHALATEUR

(30) Priority: 01.07.2022 KR 20220081058
(43) Date of publication of application: 14.02.2024
(73) Proprietor: KT&G Corporation, Daedeok-gu Daejeon 34337 (KR)
(72) Inventor: KIM, Tae Heon, Daejeon 34128 (KR); KIM, Jae Hyun, Daejeon 34128 (KR); LEE, Mi Jeong, Daejeon 34128 (KR); JEONG, Minseok, Daejeon 34128 (KR); JUNG, Yongmi, Daejeon 34128 (KR); JEOUNG, Eunmi, Daejeon 34128 (KR); CHUNG, Tae Young, Daejeon 34128 (KR); HAN, Seung Kyu, Daejeon 34128 (KR)
(74) Representative: Ter Meer Steinmeister & Partner
(86) International application number: PCT/KR2023/007450
(87) International publication number: WO 2024/005385

(56) References cited:
- WO-A1-02/45783
- WO-A1-2009/103711
- WO-A1-92/09324
- GB-A- 2 344 533
- JP-A- 2008 100 089
- JP-A- 2020 151 022
- KR-A- 20080 003 343
- KR-B1- 100 493 837
- US-A1- 2007 074 718

## Description

### Technical Field

Disclosed is an inhaler.

### Background Art

In general, an inhaler is a device used to inhale a composition such as medication through the oral cavity or nasal cavity as a liquid or gas in the process of inhalation. Such an inhaler may include a container accommodating an inhalable composition, and the composition may be sprayed from the container through a thin tube to the oral cavity or nasal cavity through an intake to be inhaled by a user.

The above description has been possessed or acquired by the inventor(s) in the course of conceiving the present disclosure and is not necessarily an art publicly known before the present application is filed.

Prior Document: Korean Patent Gazette No. 10-2021229 (Published on September 11, 2019)
WO 2009/103711 A1 relates to an actuation counter comprising a driver feature having one or more driver elements, which driver feature is arranged for movement in response to an actuation; and a count member including one or more drive receipt elements arranged thereon for receipt of drive from said one or more driver elements of the driver feature for movement of the count member. The driver feature includes one or more brake elements arranged to selectively interact with the count member to prevent overrun thereof when registering a count of an actuation. The actuation is suitably arranged for use with a drug dispenser device.
US 2007/074718 A1 relates to a medication inhaler for delivery of a medication to a patient from a canister having a valve stem, such as a MDI canister. The medication inhaler includes a chamber body having a fresh air inlet and a hollow spacer chamber. The inhaler further includes a canister retainer configured to move the canister between operational and stored positions, and an actuation lever for actuation of the valve stem to deliver a medication fluid into the hollow spacer chamber. In one example, the hollow spacer chamber tapers. In addition or alternatively, the chamber body includes a nozzle having an outlet, and a fresh air inlet having an outlet proximate the outlet of the nozzle. In addition or alternatively, the medication inhaler includes a canister adapter for accommodation of various size canisters. In addition or alternatively, the chamber body includes an inlet portion having a geometry for guiding the valve stem.
GB 2 344 533 A relates to an inhalation apparatus comprising a housing defining a socket for receiving a pressurised dispensing container. Actuator means are provided for receiving a valve stem of the dispensing container and a cylindrical chamber having an inlet and an outlet. The actuator means defines duct means to direct product dispensed from the valve stem of the dispensing container through the inlet of the cylindrical chamber in a direction substantially tangential to the major axis of the chamber. The outlet of the chamber communicates with the mouthpiece, the inlet being located at a periphery of the chamber and the outlet being located at or near a centre of the chamber such that inhalation by a user creates a cyclonic airflow in the chamber between the inlet and outlet from which the dispensed product is entrained for inhalation.
WO0245783A1 discloses an inhaler (fig.5) comprising a housing, a canister (107, fig.5) which is mounted in the housing to be replaceable; a reservoir (upstream portion 112.1 of flexible tube 111 (page 11 line 10-11), which is connected to the canister via spigot 110, kink valves, 112,121 operated by a flap vane 121 during inhalation. The drug is kept in the upper portion 112.1 until the patient inhales. An electronic counter is also foreseen in the embodiments of figures 10 and 11.
JP 2008 100089 A relates to an indicating device for a dispensing device to dispense a determined dose of medicine from a container with a valve movable between an open position and a close position. The container is reciprocally movable along the longitudinal axis line in a housing. The housing has a well capable of receiving a valve system and a discharge port. The well is communicated with a port so that a determined dose of medicine is sent out through the port when the valve is moved to the open position.
JP 2020 151022 A relates to a medicament inhalation tool comprising: a holding part for holding an actuator which changes a discharge direction of a medicament discharged from a discharge valve of an aerosol tool; a lever part for pressing the aerosol tool for driving the discharge valve; a first opening into which the medicament discharged from the aerosol tool flows; a chamber part in which the medicament flowed from the first opening is stored; and a second opening which is opened to an outflow direction having a directional component facing the inflow direction of the medicament to the fist opening, and through which the medicament stored in the chamber part exits.

### Disclosure of the Invention

### Technical Goals

An object according to an embodiment is to provide an inhaler capable of notifying a remaining amount of a composition in a canister and information on time to replace the canister by counting a number of times of filling using a vertical movement of the canister when a reservoir is filled with an inhalable composition from the canister.

The technical tasks obtainable from the present disclosure are non-limited by the above-mentioned technical tasks. And, other unmentioned technical tasks can be clearly understood from the following description by those having ordinary skill in the technical field to which the present disclosure pertains.

### Technical Solutions

An inhaler according to an embodiment for achieving the above objects includes: a housing having one surface, the other surface opposite to the one surface, and a plurality of side surfaces connecting the one surface and the other surface; a canister which is mounted on the housing to be replaceable and accommodates an inhalable composition; a reservoir which is connected to the canister so that the canister is able to be opened or closed, and stores the inhalable composition; a lever which controls an opening and closing operation of the canister; and a counter which is rotatably disposed between the canister and the lever. The counter is rotated at a predetermined angle according to the operation of the lever to notify a remaining amount of the inhalable composition in the canister.

According to an aspect, the inhaler may further include a fastening portion having one end rotatably coupled to the housing and the other end formed in a hook shape. The other end of the fastening portion may be engaged with the counter to restrict rotation of the counter in one direction

According to an aspect, the inhaler may further include: a first counter member formed in a circular shape and having a center rotatably coupled to the inside of the housing; and a plurality of second counter members each formed in a sawtooth shape and arranged at regular intervals around a circumference of the first counter member. The first counter member may be rotated when the canister is opened.

According to an aspect, the lever may include: a support member which extends in a first direction toward the canister; and a protrusion member which is rotatably coupled to the support member around a rotation shaft provided in the support member, and when the lever is pressed, the protrusion member may apply a pressing force to one of the plurality of second counter members in the first direction to rotate the first counter member.

According to an aspect, when the lever is pressed in the first direction, the support member may restrict rotation of the protrusion member, and when the lever returns to an original position, the support member may allow the rotation of the protrusion member.

According to an aspect, the protrusion member may be disposed so that rotation in a direction toward the canister is not restrained and rotation in a direction away from the canister is restrained in a state where an end portion stands toward the one surface of the housing.

According to an aspect, the inhaler may further include a reset portion provided between the fastening portion and the counter. The reset portion may separate the fastening portion from the counter when the canister is removed from the housing.

According to an aspect, the counter may further include an elastic member provided on the first counter member, and the elastic member may be compressed along a rotation direction of the first counter member by the lever, and rotate the first counter member to an original position when the canister is removed from the housing.

According to an aspect, when the lever is pressed, the canister may be opened to allow movement of the inhalable composition to the reservoir, and when the lever is not pressed, the canister may be closed.

According to an aspect, the first counter member may display different colors or a series of numbers on one surface along a circumferential direction.

According to an aspect, the housing may include a display window formed on one side, and the display window may expose a portion of the counter showing the remaining amount to the outside.

According to an aspect, the reservoir may be formed of a transparent material, the housing may further include a viewing window formed at a position where the reservoir is disposed, and the viewing window may expose an amount of the inhalable composition in the reservoir to the outside.

### Effects

According to the inhaler according to an embodiment, there is an effect that the remaining amount of the composition in the canister and information on time to replace the canister may be notified by counting the number of times of filling using a vertical movement of the canister when the reservoir is filled with an inhalable composition from the canister.

The effects of the inhaler are not limited to the above-mentioned effects, and other unmentioned effects can be clearly understood from the following description by one of ordinary skill in the art to which the present disclosure pertains.

### Brief Description of Drawings

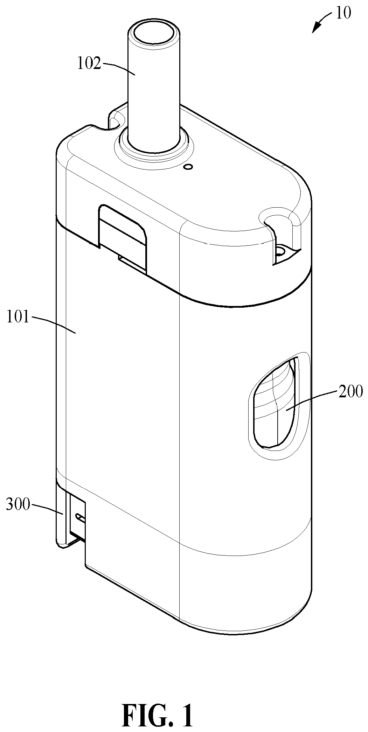

FIG. 1 is a perspective view of a front side of an inhaler according to an embodiment.

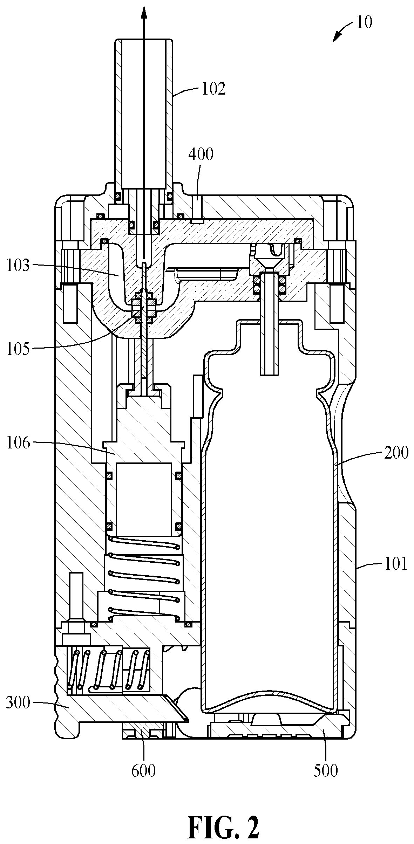

FIG. 2 is a cross-sectional view of an inhaler according to an embodiment.

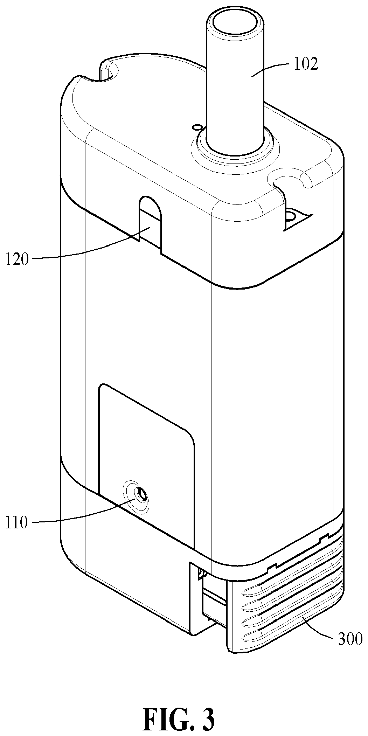

FIG. 3 is a perspective view of a rear side of an inhaler according to an embodiment.

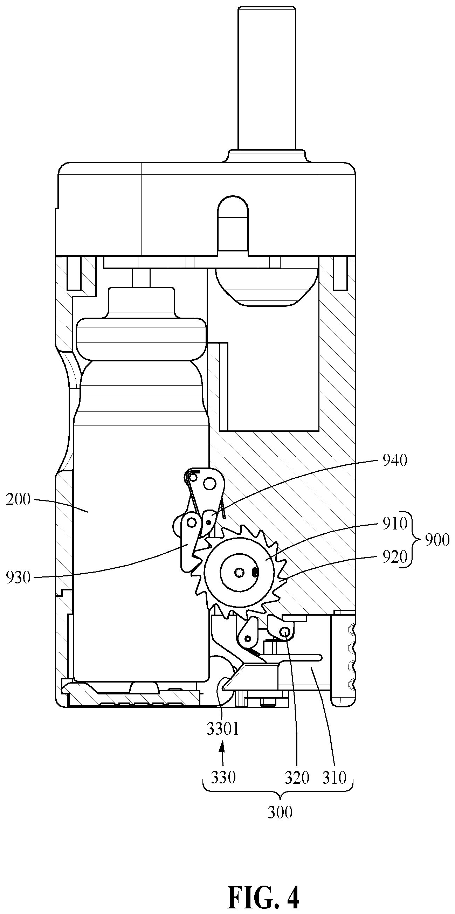

FIG. 4 illustrates a counter that operates in association with a lever.

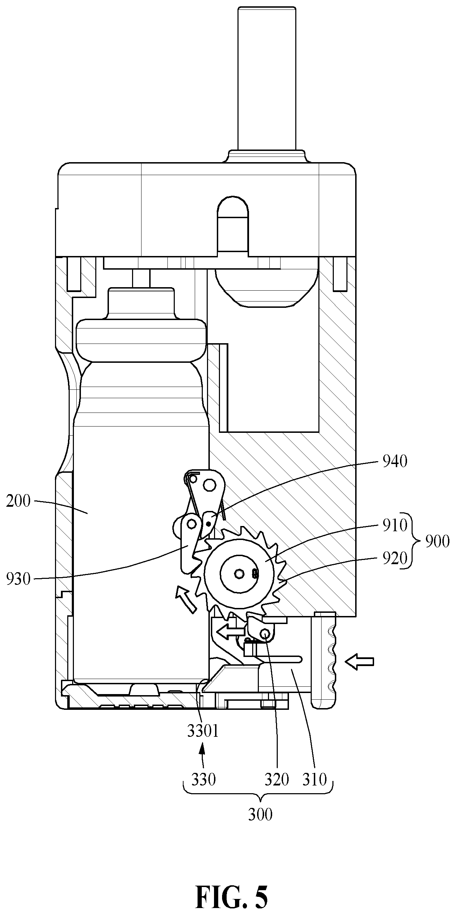

FIG. 5 illustrates a counter that rotates when a lever is pressed.

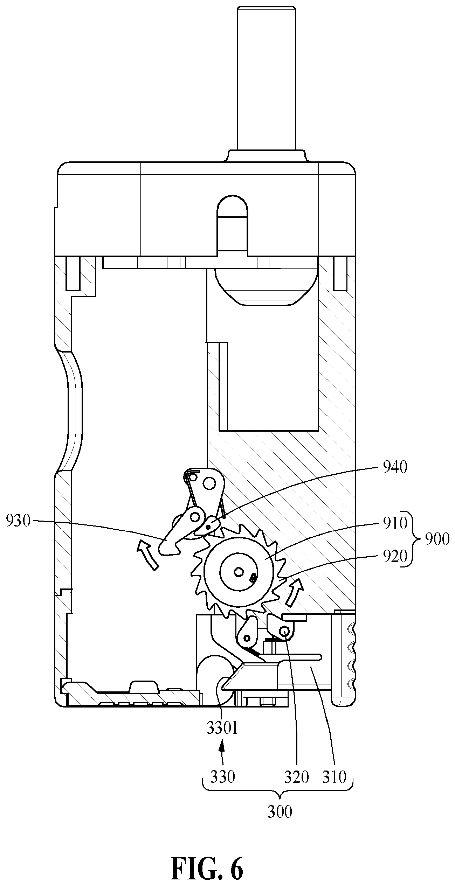

FIG. 6 illustrates a counter that returns to an original position when a canister is removed.

The accompanying drawings illustrate desired embodiments of the present disclosure and are provided together with the detailed description for better understanding of the technical idea of the present disclosure. Therefore, the present disclosure should not be construed as being limited to the embodiments set forth in the drawings.

### Best Mode for Carrying Out the Invention

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Further, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe constituent elements of the embodiments. These terms are used only for the purpose of discriminating one constituent element from another constituent element, and the nature, the sequences, or the orders of the constituent elements are not limited by the terms. When one constituent element is described as being "connected", "coupled", or "attached" to another constituent element, it should be understood that one constituent element can be connected or attached directly to another constituent element, and an intervening constituent element can also be "connected", "coupled", or "attached" to the constituent elements.

The same name may be used to describe an element included in the embodiments described above and an element having a common function. Unless otherwise mentioned, the descriptions on the embodiments may be applicable to the following embodiments and thus, duplicated descriptions will be omitted for conciseness.

FIG. 1 is a perspective view of a front side of an inhaler 10 according to an embodiment.

FIG. 2 is a cross-sectional view of the inhaler 10 according to an embodiment.

FIG. 3 is a perspective view of a rear side of the inhaler 10 according to an embodiment.

FIG. 4 illustrates a counter 900 that operates in association with a lever 300.

FIG. 5 illustrates the counter 900 that rotates when the lever 300 is pressed.

FIG. 6 illustrates the counter 900 that returns to an original position when a canister 200 is removed.

Referring to FIG. 1, the inhaler 10 according to an embodiment may include a housing 101, a mouthpiece 102, a reservoir 103, the canister 200, and the lever 300.

The housing 101 may include a first surface formed on one surface, a second surface opposite to the first surface, and a plurality of side surfaces connecting the first surface and the second surface. The first surface of the housing 101 may be, for example, a surface located on the top of the housing 101, and the second surface may be, for example, a bottom surface of the housing 101.

The mouthpiece 102 may be disposed on the first surface of the housing 101. A user may inhale an inhalable composition accommodated in the inhaler 10 through the mouthpiece 102. At this time, the user may inhale the composition, for example, in the form of an aerosol or in the form of a powder. Hereinafter, the inhaler 10 according to an embodiment will be described using the inhaler 10 that sprays an inhalable composition in the form of an aerosol as an example.

Referring to FIGS. 1 and 2, a reservoir 103 may be disposed inside the housing 101. Since one side of the canister 200 is connected to the reservoir 103, the inhalable composition accommodated in the canister 200 may be moved to and stored in the reservoir 103. In addition, as the user applies a suction force through the mouthpiece 102, the inhalable composition stored in the reservoir 103 may be discharged through the mouthpiece 102 in the form of an aerosol, and accordingly, the inhalable composition stored in the reservoir 103 may be gradually consumed.

The canister 200 may be mounted inside the housing 101. In this case, the canister 200 may be mounted interchangeably inside the housing 101. In addition, the canister 200 may accommodate an inhalable composition therein. A certain amount of the composition of the canister 200 may be filled in the reservoir 103.

The lever 300 may be pressed by the user to fill the inside of the reservoir 103 with the composition accommodated in the canister 200. The lever 300 may form one side surface of the housing 101 and may be positioned adjacent to the second surface. When the user presses the lever 300, the lever 300 may push up a bottom surface of the canister 200 so that an injection hole 220 of the canister 200 communicates with the reservoir 103, and the composition may move from the canister 200 to the reservoir 103 through the injection hole 220.

Hereinafter, a direction from one side surface of the housing 101 where the lever 300 is located to the canister 200 is defined as a first direction, and a direction from the second surface of the housing 101 to the first surface is defined as a second direction.

Referring to FIG. 3, in the inhaler 10 according to an embodiment, the user may visually identify a remaining amount of the composition stored in the reservoir 103 through a viewing window 120 provided on the housing 101. In addition, the inhaler 10 according to an embodiment may include the counter 900 that counts the number of times of filling in connection with a vertical movement of the canister 200 during the filling of the reservoir 103, and a display window 110 that displays a remaining amount of the composition in the canister 200. The composition stored in the reservoir 103 may be sprayed in the form of an aerosol as the user applies a suction force to the mouthpiece 102. At this time, an inhalation interlocking valve 105 that opens and closes the reservoir 103 by the suction force may operate, and the opening and closing operation of the inhalation interlocking valve 105 may be controlled by a piston 106. Meanwhile, a relief valve (not shown) may be provided on the reservoir 103, and a relief vent hole 400 may be provided on the first surface of the housing 101. The relief valve is interlocked with the lever 300 through a relief bar (not shown) or a relief bar movement protrusion (not shown), and accordingly, the relief valve may be opened first to discharge a residual gas in the reservoir 103, before the lever 300 pushes the canister 200 up to fill the reservoir 103. The inhaler 10 according to an embodiment may further include a cover 500 and a locking device 600 for preventing the canister 200 from being separated.

As described above, in order to notify the time to replace the canister 200, the inhaler 10 according to an embodiment described above needs to deliver information on the remaining amount of the composition in the canister 200 to the user. Accordingly, in the inhaler 10 according to an embodiment, a counter function for notifying the available number of filling to the user by counting the number of filling of the filling lever 300 is applied. The counter 900 having such a function may calculate the number of filling in consideration of a total amount of the composition and a volume of the reservoir 103 and estimate the remaining number of filling by counting the number for each filling, and a counting method of mechanically operating in association with the operation of the filling lever 300 may be applied.

Referring to FIG. 4, the counter 900 of the inhaler 10 according to an embodiment may be rotatably provided on one of the plurality of side surfaces of the housing 101. In this case, the counter 900 may be disposed between the canister 200 and the lever 300. The counter 900 may be rotatably provided in association with the lever 300. For example, the counter 900 may be rotated at a predetermined angle according to the operation of the lever 300 to notify the user of the remaining amount of the inhalable composition in the canister 200 by the color or number.

Specifically, the counter 900 may include a first counter member 910 and a second counter member 920.

The first counter member 910 may be formed in a circular shape and may have a center that is rotatably coupled to the inside of the housing 101. A symbol indicating the remaining amount of the canister 200 may be marked on one surface of the first counter member 910. For example, different colors or a series of numbers may be displayed on one surface of the first counter member 910 along a circumferential direction.

A plurality of second counter members 920 may be provided, and they may be formed in a sawtooth shape and arranged at regular intervals around the circumference of the first counter member 910.

The second counter members 920 may be engaged with the lever 300, and therefore, when the canister 200 is opened, the second counter members 920 may be moved in the circumferential direction and the first counter member 910 may be rotated.

At this time, the inhaler 10 according to an embodiment may further include a fastening portion 930 to prevent reverse rotation of the first counter member 910 other than the rotation by the lever 300.

The fastening portion 930 may have one end rotatably coupled to the housing 101 and the other end formed in a hook shape. The other end of the fastening portion 930 may be engaged with the second counter member 920. Accordingly, the fastening portion 930 may restrain the rotation of the first counter member 910 in one direction. That is, the fastening portion 930 may allow the rotation of the second counter member 920 in a direction toward the canister 200 by the lever 300, but may not allow the rotation of the second counter member 920 in a direction away from the canister 200.

As described above, when the lever 300 of the inhaler 10 according to an embodiment is pressed by the user, the canister 200 may be opened so that the reservoir 103 is filled with the inhalable composition. At this time, since the lever 300 and the counter 900 are interlocked with each other, the counter 900 may be rotated by the operation of the lever 300 to notify the remaining amount of the canister 200. When the lever 300 is pressed by the user, the lever 300 may be moved in the first direction and the lever 300 may move the canister 200 in the second direction and rotate the counter 900 at the same time.

Referring to FIGS. 4 and 5, the lever 300 may include a support member 310 and a protrusion member 320.

The support member 310 may extend in the first direction toward the canister 200.

The protrusion member 320 may be rotatably coupled to the support member 310 around a rotation shaft provided in the support member 310. The protrusion member 320 may have an end portion protruding in a radial direction from the rotation shaft at one side. The end portion of the protrusion member 320 may be disposed in a standing state toward the first surface of the housing 101.

When the lever 300 is pressed, the protrusion member 320 may be moved in the first direction and may apply a pressing force to one of the plurality of second counter members 920 in the first direction. Accordingly, the second counter member 920 may be rotated in a direction toward the canister 200, thereby rotating the first counter member 910.

As described above, when the lever 300 is pressed in the first direction, the support member 310 may restrain the rotation of the protrusion member 320. That is, the rotation of the protrusion member 320 in a direction away from the canister 200 may be restrained in a state where the end portion stands toward the first surface of the housing 101.

Therefore, when the lever 300 is pressed, the protrusion member 320 also comes into contact with the second counter member 920 as it moves in the first direction. At this time, the protrusion member 320 may not be rotated but push the second counter member 920 to be rotated in a direction toward the canister 200.

As a result, when the lever 300 opens the canister 200, the counter 900 may be rotated to notify the changed remaining amount of the composition in the canister 200.

Meanwhile, when the lever 300 returns to its original position, the support member 310 may allow the rotation of the protrusion member 320. That is, the rotation of the protrusion member 320 in the direction toward the canister 200 may not be restrained in a state where the end portion stands up toward the first surface of the housing 101.

Accordingly, when the lever 300 moves in a direction opposite to the first direction, the protrusion member 320 may return to its original position without rotating the second counter member 920.

When the refilling of the composition in the reservoir 103 is repeated a certain number of times, the counter 900 may notify that there is no remaining composition in the canister 200, and the user who has confirmed it may remove the canister 200 from the housing 101.

Referring to FIG. 6, when the canister 200 is removed from the housing 101, the first counter member 910 may be rotated to return to its initial position. At this time, in order to return to the initial position, the counter 900 may rotate in a direction opposite to a rotation direction by the lever 300. To allow such reverse rotation, the inhaler 10 according to an embodiment may further include a reset portion 940.

The reset portion 940 may be disposed between the fastening portion 930 and the counter 900. The reset portion 940 may have one end rotatably mounted on the inside the housing 101 and the other end in contact with the fastening portion 930. When the canister 200 is removed from the housing 101, the reset portion 940 may push and rotate the fastening portion 930 in a direction toward the canister 200. Accordingly, the hook portion of the fastening portion 930 that is engaged with the second counter member 920 may be separated from the second counter member 920.

In addition, the counter 900 may further include an elastic member (not shown) provided on the first counter member 910.

When the first counter member 910 is rotated by the lever 300, the elastic member may be compressed along the rotation direction of the counter 900. When the rotational restriction does not act for the counter 900, for example, when the canister 200 is removed from the housing 101 to remove the rotational restriction by the fastening portion 930, the elastic member may rotate the first counter member 910 to its original position.

Referring back to FIG. 3, the housing 101 of the inhaler 10 according to an embodiment may further include a display window, and the remaining amount in the canister 200 displayed on the counter 900 may be notified externally through the display window 110 so that the user may confirm it.

The display window 110 may be formed of a hole or a transparent material formed on one side of the housing 101. The display window 110 may be formed at a position corresponding to the first counter member 910 of the counter 900. The display window 110 may expose a color or number displayed by the first counter member 910 to the outside.

Meanwhile, in order to confirm the remaining amount of the composition in the reservoir 103, the reservoir 103 of the inhaler 10 according to an embodiment may be formed of a transparent material, and the housing 101 may further include the viewing window 120. The viewing window 120 may be a hole formed on one side of the housing 101 and may be formed at a position corresponding to the reservoir 103. This viewing window 120 may expose the amount of inhalable composition in the reservoir 103 to the outside. Accordingly, the user may easily check the remaining amount and whether the reservoir 103 is completely filled.

Since the counter 900 interlocked with the filling lever 300 is applied to the inhaler 10 according to an embodiment described above, the number of filling may be counted by using the vertical movement of the canister 200 when the reservoir 103 is filled with the inhalable composition from the canister 200, thereby notifying the remaining amount of the composition in the canister 200 and the information on the time to replace the canister 200.

## Claims

1. An inhaler (10) comprising:
a housing (101) having one surface, the other surface opposite to the one surface, and a plurality of side surfaces connecting the one surface and the other surface;
a canister (200) which is mounted on the housing (101) to be replaceable and accommodates an inhalable composition;
a reservoir (103) which is connected to the canister (200) so that the canister (200) is able to be opened or closed, and stores the inhalable composition;
a lever (300) which controls an opening and closing operation of the canister (200); and
a counter (900) which is rotatably disposed between the canister (200) and the lever (300),
wherein the counter (900) is rotated at a predetermined angle according to the operation of the lever (300) to notify a remaining amount of the inhalable composition in the canister (200).

2. The inhaler of claim 1, further comprising:
a fastening portion (930) having one end rotatably coupled to the housing (101) and the other end formed in a hook shape,
wherein the other end of the fastening portion (930) is engaged with the counter (900) to restrict rotation of the counter (900) in one direction.

3. The inhaler of claim 1,
wherein the counter (900) comprises:
a first counter member (910) formed in a circular shape and having a center rotatably coupled to the inside of the housing (101); and
a plurality of second counter members (920) each formed in a sawtooth shape and arranged at regular intervals around a circumference of the first counter member (910), and
wherein the first counter member (910) is rotated when the canister (200) is opened.

4. The inhaler of claim 3,
wherein the lever (300) comprises:
a support member (310) which extends in a first direction toward the canister (200); and
a protrusion member (320) which is rotatably coupled to the support member (310) around a rotation shaft provided in the support member (310), and
wherein, when the lever (300) is pressed, the protrusion member (320) applies a pressing force to one of the plurality of second counter members (920) in the first direction to rotate the first counter member (910).

5. The inhaler of claim 4,
wherein, when the lever (300) is pressed in the first direction, the support member (310) restricts rotation of the protrusion member (320), and
wherein, when the lever (300) returns to an original position, the support member (310) allows the rotation of the protrusion member (320).

6. The inhaler of claim 4, wherein the protrusion member (320) is disposed so that rotation in a direction toward the canister (200) is not restrained and rotation in a direction away from the canister (200) is restrained in a state where an end portion stands toward the one surface of the housing (101).

7. The inhaler of claim 2, further comprising:
a reset portion (940) provided between the fastening portion (930) and the counter (900),
wherein the reset portion (940) separates the fastening portion (930) from the counter (900) when the canister (200) is removed from the housing (101).

8. The inhaler of claim 3,
wherein the counter (900) further comprises an elastic member provided on the first counter member (910), and
wherein the elastic member is compressed along a rotation direction of the first counter member (910) by the lever (300), and rotates the first counter member (910) to an original position when the canister (200) is removed from the housing (101).

9. The inhaler of claim 1, wherein, when the lever (300) is pressed, the canister (200) is opened to allow movement of the inhalable composition to the reservoir (103), and when the lever (300) is not pressed, the canister (200) is closed.

10. The inhaler of claim 3, wherein the first counter member (910) displays different colors or a series of numbers on one surface along a circumferential direction.

11. The inhaler of claim 1,
wherein the housing (101) comprises a display window (110) formed on one side, and
wherein the display window (110) exposes a portion of the counter (900) showing the remaining amount to the outside.

12. The inhaler of claim 1,
wherein the reservoir (103) is formed of a transparent material,
wherein the housing (101) further comprises a viewing window (110) formed at a position where the reservoir (103) is disposed, and
wherein the viewing window (110) exposes an amount of the inhalable composition in the reservoir (103) to the outside.

## Patentansprüche

1. Inhalator (10), der Folgendes umfasst:
ein Gehäuse (101), das eine Oberfläche, die andere Oberfläche, die der einen Oberfläche gegenüberliegt, und mehrere Seitenflächen, die die eine Oberfläche und die andere Oberfläche verbinden, aufweist;
einen Kanister (200), der an dem Gehäuse (101) austauschbar montiert ist und eine inhalierbare Zusammensetzung fasst;
ein Reservoir (103), das mit dem Kanister (200) derart verbunden ist, dass der Kanister (200) geöffnet oder geschlossen werden kann, und die inhalierbare Zusammensetzung speichert;
einen Hebel (300), der einen Öffnungs- und Schließvorgang des Kanisters (200) steuert; und
einen Zähler (900), der zwischen dem Kanister (200) und dem Hebel (300) drehbar angeordnet ist,
wobei der Zähler (900) entsprechend der Betätigung des Hebels (300) um einen vorgegebenen Winkel gedreht wird, um eine verbleibende Menge der inhalierbaren Zusammensetzung in dem Kanister (200) anzuzeigen.

2. Inhalator nach Anspruch 1, der ferner Folgendes umfasst:
einen Befestigungsabschnitt (930), wovon ein Ende drehbar mit dem Gehäuse (102) gekoppelt ist und sein anderes Ende hakenförmig ausgebildet ist,
wobei sich das andere Ende des Befestigungsabschnitts (930) mit dem Zähler in Eingriff (900) befindet, um die Drehung des Zählers (900) in eine Richtung zu begrenzen.

3. Inhalator nach Anspruch 1,
wobei der Zähler (900) Folgendes umfasst:
ein erstes Zählerelement (910), das kreisförmig gebildet ist und einen Mittelpunkt aufweist, der drehbar mit der Innenseite des Gehäuses (101) gekoppelt ist; und
mehrere zweite Zählerelemente (920), die jeweils sägezahnförmig gebildet sind und in regelmäßigen Intervallen um einen Umfang des ersten Zählerelements (910) angeordnet sind, und
wobei das erste Zählerelement (910) gedreht wird, wenn der Kanister (200) geöffnet wird.

4. Inhalator nach Anspruch 3,
wobei der Hebel (300) Folgendes umfasst:
ein Tragelement (310), das sich in einer ersten Richtung zu dem Kanister (200) hin erstreckt; und
ein Vorsprungelement (320), das mit dem Tragelement (310) drehbar um eine Drehwelle, die in dem Tragelement (310) vorgesehen ist, gekoppelt ist, und
wobei dann, wenn der Hebel (300) gedrückt wird, das Vorsprungelement (320) in der ersten Richtung eine Druckkraft auf eines der mehreren zweiten Zählerelemente (920) ausübt, um das erste Zählerelement (910) drehen.

5. Inhalator nach Anspruch 4,
wobei dann, wenn der Hebel (300) in der ersten Richtung gedrückt wird, das Tragelement (310) die Drehung des Vorsprungelements (320) begrenzt, und
wobei dann, wenn der Hebel (300) in eine ursprüngliche Position zurückkehrt, das Tragelement (310) die Drehung des Vorsprungelements (320) erlaubt.

6. Inhalator nach Anspruch 4, wobei das Vorsprungelement (320) derart angeordnet ist, dass eine Drehung in einer Richtung zu dem Kanister (200) hin nicht begrenzt ist und in einem Zustand, in dem ein Endabschnitt zu der einen Oberfläche des Gehäuses (101) hin steht, eine Drehung in einer Richtung von dem Kanister (200) weg begrenzt ist.

7. Inhalator nach Anspruch 2, der ferner Folgendes umfasst:
einen Rückstellabschnitt (940), der zwischen dem Befestigungsabschnitt (930) und dem Zähler (900) vorgesehen ist,
wobei der Rückstellabschnitt (940) den Befestigungsabschnitt (930) von dem Zähler (900) trennt, wenn der Kanister (200) aus dem Gehäuse (101) entfernt wird.

8. Inhalator nach Anspruch 3,
wobei der Zähler (900) ferner ein elastisches Element umfasst, das auf dem ersten Zählerelement (910) vorgesehen ist, und
wobei das elastische Element entlang einer Drehrichtung des ersten Zählerelements (910) durch den Hebel (300) komprimiert wird und das erste Zählerelement (910) in einer ursprüngliche Position dreht, wenn der Kanister (200) von dem Gehäuse (101) entfernt wird.

9. Inhalator nach Anspruch 1, wobei dann, wenn der Hebel (300) gedrückt wird, der Kanister (200) geöffnet wird, um eine Bewegung der inhalierbaren Zusammensetzung zu dem Reservoir (103) zu erlauben, und dann, wenn der Hebel (300) nicht gedrückt wird, der Kanister (200) geschlossen ist.

10. Inhalator nach Anspruch 3, wobei das erste Zählerelement (910) unterschiedliche Farben oder eine Reihe von Zahlen auf einer Oberfläche entlang einer Umfangsrichtung anzeigt.

11. Inhalator nach Anspruch 1,
wobei das Gehäuse (101) ein Anzeigefenster (110) umfasst, das auf der einen Seite gebildet ist, und
wobei das Anzeigefenster (110) einen Abschnitt des Zählers (900), der die verbleibende Menge anzeigt, nach außen freilegt.

12. Inhalator nach Anspruch 1,
wobei das Reservoir (103) aus einem durchsichtigen Material gebildet ist,
wobei das Gehäuse (101) ferner ein Sichtfenster (110) aufweist, das an einer Position gebildet ist, an der das Reservoir (103) angeordnet ist, und
wobei das Sichtfenster (110) eine Menge der inhalierbaren Zusammensetzung in dem Reservoir (103) nach außen sichtbar macht.

## Revendications

1. Inhalateur (10) comportant :
un boîtier (101) ayant une première surface, la seconde surface étant opposée à la première surface, et une pluralité de surfaces latérales reliant la première surface et la seconde surface ;
une cartouche (200) qui est montée sur le boîtier (101) de manière à pouvoir être remplacée et qui reçoit une composition inhalable ;
un réservoir (103) qui est relié à la cartouche (200) de sorte que la cartouche (200) peut être ouverte ou fermée, et stocke la composition inhalable ;
un levier (300) qui commande une opération d'ouverture et de fermeture de la cartouche (200) ; et
un compteur (900) qui est disposé de manière rotative entre la cartouche (200) et le levier (300),
dans lequel le compteur (900) est tourné à un angle prédéterminé en fonction de l'actionnement du levier (300) pour notifier une quantité restante de la composition inhalable dans la cartouche (200).

2. Inhalateur selon la revendication 1, comportant en outre :
une partie de fixation (930) ayant une première extrémité couplée en rotation au boîtier (101) et la seconde extrémité formée en une forme de crochet,
dans lequel la seconde extrémité de la partie de fixation (930) vient en prise avec le compteur (900) pour restreindre une rotation du compteur (900) dans un sens.

3. Inhalateur selon la revendication 1,
dans lequel le compteur (900) comporte :
un premier élément de compteur (910) formé en une forme circulaire et ayant un centre couplé en rotation à l'intérieur du boîtier (101) ; et
une pluralité de seconds éléments de compteur (920) chacun formés en une forme de dents de scie et agencés à des intervalles réguliers autour d'une circonférence du premier élément de compteur (910), et
dans lequel le premier élément de compteur (910) tourne lorsque la cartouche (200) est ouverte.

4. Inhalateur selon la revendication 3,
dans lequel le levier (300) comporte :
un élément de support (310) qui s'étend dans une première direction vers la cartouche (200) ; et
un élément de saillie (320) qui est couplé en rotation à l'élément de support (310) autour d'un arbre de rotation agencé dans l'élément de support (310), et
dans lequel, lorsque le levier (300) est pressé, l'élément de saillie (320) applique une force de pression à un élément de la pluralité de seconds éléments de compteur (920) dans la première direction pour faire tourner le premier élément de compteur (910).

5. Inhalateur selon la revendication 4,
dans lequel, lorsque le levier (300) est pressé dans la première direction, l'élément de support (310) restreint une rotation de l'élément de saillie (320), et
dans lequel, lorsque le levier (300) revient à une position d'origine, l'élément de support (310) permet la rotation de l'élément de saillie (320).

6. Inhalateur selon la revendication 4, dans lequel l'élément de saillie (320) est disposé de sorte qu'une rotation dans un sens allant vers la cartouche (200) n'est pas restreinte et une rotation dans un sens s'éloignant de la cartouche (200) est restreinte dans un état où une partie d'extrémité se trouve vers la première surface du boîtier (101).

7. Inhalateur selon la revendication 2, comportant en outre :
une partie de rappel (940) agencée entre la partie de fixation (930) et le compteur (900),
dans lequel la partie de rappel (940) sépare la partie de fixation (930) du compteur (900) lorsque la cartouche (200) est retirée du boîtier (101).

8. Inhalateur selon la revendication 3,
dans lequel le compteur (900) comporte en outre un élément élastique agencé sur le premier élément de compteur (910), et
dans lequel l'élément élastique est comprimé le long d'un sens de rotation du premier élément de compteur (910) par le levier (300), et fait tourner le premier élément de compteur (910) jusqu'à une position d'origine lorsque la cartouche (200) est retirée du boîtier (101).

9. Inhalateur selon la revendication 1, dans lequel, lorsque le levier (300) est pressé, la cartouche (200) est ouverte pour permettre un mouvement de la composition inhalable jusqu'au réservoir (103), et lorsque le levier (300) n'est pas pressé, la cartouche (200) est fermée.

10. Inhalateur selon la revendication 3, dans lequel le premier élément de compteur (910) affiche différentes couleurs ou une série de nombres sur une surface le long d'une direction circonférentielle.

11. Inhalateur selon la revendication 1,
dans lequel le boîtier (101) comporte une fenêtre d'observation (110) formée sur un côté, et
dans lequel la fenêtre d'affichage (110) expose une partie du compteur (900) indiquant la quantité restante à l'extérieur.

12. Inhalateur selon la revendication 1,
dans lequel le réservoir (103) est formé d'un matériau transparent,
dans lequel le boîtier (101) comporte en outre une fenêtre d'observation (110) formée à une position où le réservoir (103) est disposé, et
dans lequel la fenêtre de visualisation (110) expose une quantité de la composition inhalable dans le réservoir (103) à l'extérieur.
